(19) Europäisches Patentamt
European Patent Office
Office européen des brevets

(11) Veröffentlichungsnummer: **0 040 310**
**A2**

(12) # EUROPÄISCHE PATENTANMELDUNG

(21) Anmeldenummer: 81102487.6

(22) Anmeldetag: 02.04.81

(51) Int. Cl.[3]: **C 07 D 521/00**
A 01 N 43/00
//(C07D521/00, 277/16, 235/28, 263/58, 277/74, 233/84, 233/42, 249/12, 285/12, 271/10, 239/38, 239/10, 277/76, 417/12, 413/12, 405/12)

(30) Priorität: 12.04.80 DE 3014157

(43) Veröffentlichungstag der Anmeldung: 25.11.81 Patentblatt 81/47

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt/Main 80(DE)

(72) Erfinder: Koch, Manfred, Dr.
Kreuzheck 2
D-6239 Eppstein/Taunus(DE)

(72) Erfinder: Mildenberger, Hilmar, Dr.
Fasanenstrasse 24
D-6233 Kelkheim (Taunus)(DE)

(72) Erfinder: Sachse, Burkhard, Dr.
An der Ziegelei 30
D-6233 Kelkheim (Taunus)(DE)

(54) **Fungizide, heterocyclisch substituierte Thioglykolsäureanilide, Verfahren zu ihrer Herstellung und diese enthaltende Schädlingsbekämpfungsmittel.**

(57) Gegenstand der Erfindung sind neue heterocyclisch substituierte Thioglykolsäureanilide der allgemeinen Formel I,

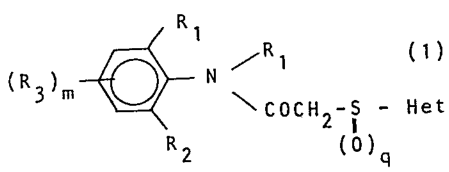
(1)

worin

R= 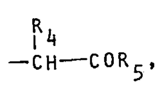, 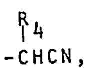, 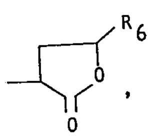,

$R_1$ = $(C_1 - C_3)$-Alkyl, Halogen, $(C_1 - C_3)$-Alkoxy, $(C_1 - C_2)$-Alkylthio, $(C_2 - C_4)$-Alkenyl,
$R_2$ = Wasserstoff, $(C_1 - C_2)$-Alkyl,
$R_3$ = Methyl, Halogen,
$R_4$, $R_6$ = Wasserstoff, Methyl,
$R_5$ = Hydroxy, $(C_1 - C_4)$-Alkoxy, $(C_1 - C_3)$-Alkylthio, $(C_1 - C_2)$-Alkoxyäthoxy, Amino, $(C_1 - C_2)$-Alkylamino, Di-$(C_1 - C_2)$-alkylamino, O-Kat, wobei Kat für ein Kationenäquivalent einer anorganischen oder organischen Base steht,
Het = ein 5 - 6 gliedriger, gesättigter oder ungesättigter Heterocyclus, gegebenenfalls benzokondensiert, mit bis zu 4 Heteroatomen aus der Gruppe N, O, S, wie z.B.

./...

EP 0 040 310 A2

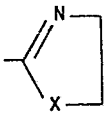 , 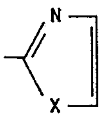 , 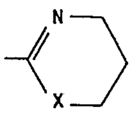 , 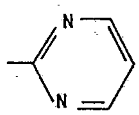 ,

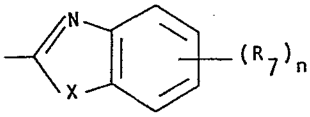 , 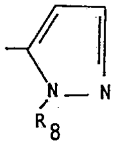 , 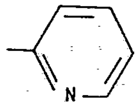 ,

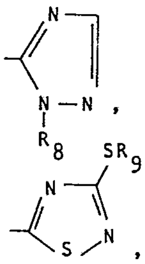 , 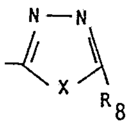 , 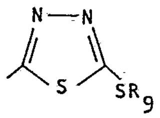 , 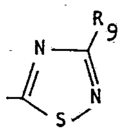 ,

wobei jeder Heterocyclus zusätzlich im Kohlenstoffteil eine oder mehrere ($C_1$-$C_4$)-Alkylgruppen tragen kann, und

X = O, S, $NR_8$,
$R_8$ = Wasserstoff, ($C_1$ - $C_4$)-Alkyl,
$R_7$ = ($C_1$ - $C_4$)-Alkyl, Halogen,
$R_9$ = ($C_1$ - $C_4$)-Alkyl,
n = 0, 1,
m = 0, 1, 2,
q = 0, 1, 2
bedeuten,

ferner Verfahren zu deren Herstellung sowie deren Verwendung als Schädlingsbekämpfungsmittel.

**Fungizide, heterocyclisch substituierte Thioglykolsäureanilide, Verfahren zu ihrer Herstellung und diese enthaltende Schädlingsbekämpfungsmittel**

Gegenstand der vorliegenden Erfindung sind neue, fungizide, heterocyclisch substituierte Thioglykolsäureanilide, Verfahren zu ihrer Herstellung sowie ihre Verwendung als Schädlingsbekämpfungsmittel, insbesondere im Pflanzenschutz.

Die erfindungsgemäßen Verbindungen besitzen die allgemeine Formel I,

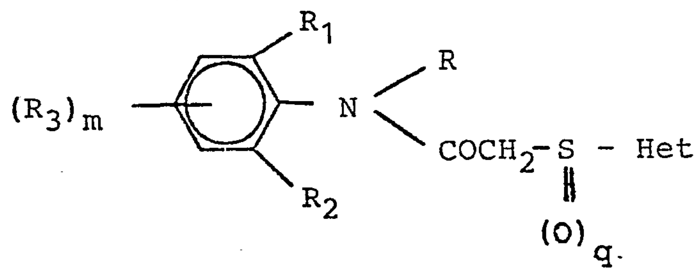

worin

R = 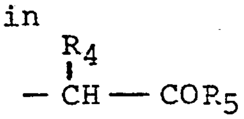 , 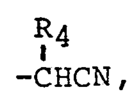 , 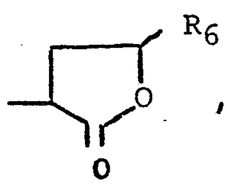 ,

$R_1$ = $(C_1 - C_3)$-Alkyl, Halogen, $(C_1 - C_3)$-Alkoxy, $(C_1 - C_2)$-Alkylthio, $(C_2 - C_4)$-Alkenyl,

$R_2$ = Wasserstoff, $(C_1 - C_2)$-Alkyl,

$R_3$ = Methyl, Halogen,

$R_4$, $R_6$ = Wasserstoff, Methyl,

$R_5$ = Hydroxy, $(C_1 - C_4)$-Alkoxy, $(C_1 - C_3)$-Alkylthio, $(C_1 - C_2)$-Alkoxyäthoxy, Amino, $(C_1 - C_2)$-Alkylamino, Di-$(C_1 - C_2)$-alkylamino, O-Kat, wobei Kat für ein Kationenäquivalent einer anorganischen oder organischen Base steht, vorzugsweise Na, K, 1/2 Ca, Ammonium,

Het = ein 5 - 6 gliedriger, gesättigter oder ungesättigter Heterocyclus, gegebenenfalls benzokondensiert, mit bis zu 4 Heteroatomen aus der Gruppe N, O, S, vorzugsweise ein Heterocyclus aus der nachfolgend genannten Reihe:

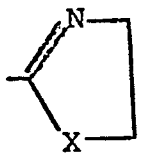 , 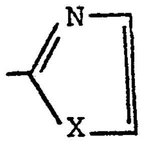 , 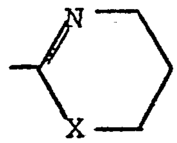 , 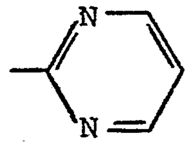 ,

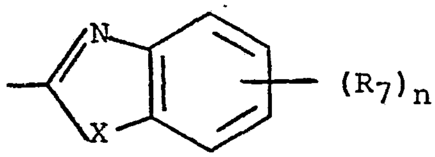 , 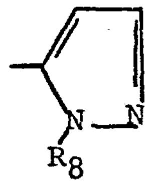 , 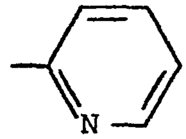 ,

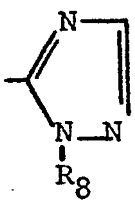 , 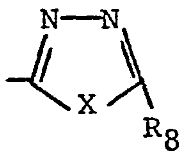 , 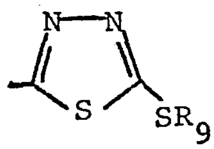 , 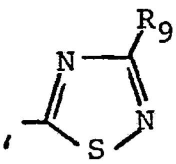 ,

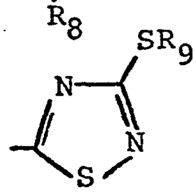 ,

wobei jeder Heterocyclus zusätzlich im Kohlenstoffteil eine oder mehrere $(C_1 - C_4)$-Alkylgruppen tragen kann, und

X = O, S, $NR_8$,

$R_8$= Wasserstoff, $(C_1 - C_4)$-Alkyl,

$R_7$= $(C_1 - C_4)$-Alkyl, Halogen,

$R_9$= $(C_1 - C_4)$-Alkyl,

n = 0, 1,

m = 0, 1, 2,

q = 0, 1, 2

bedeuten.

Bevorzugte Verbindungen der Formel I sind solche, in denen

R = 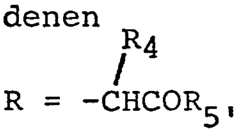 , 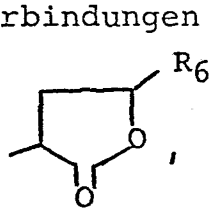 ,

$R_1$= Methyl, Äthyl, Isopropyl, Chlor, Brom,

$R_2$, $R_3$ = Methyl,

$R_4$, $R_6$ = Wasserstoff, Methyl,

$R_5$ = Hydroxy, $(C_1 - C_2)$-Alkoxy, $(C_1 - C_2)$-Alkylthio,

Het = ein 5 - 6 gliedriger, gesättigter oder ungesättigter Heterocyclus mit bis zu 3 Heteroatomen aus der Reihe N, O, S, vorzugsweise ein gegebenenfalls im Kohlenstoffteil ein- bis zweifach $(C_1 - C_4)$-alkylsubstituierter Heterocyclus der nachfolgend genannten Reihe:

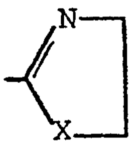 , 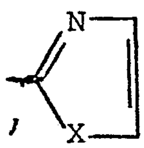 , 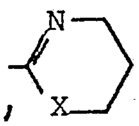 , 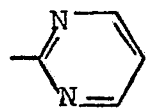 , 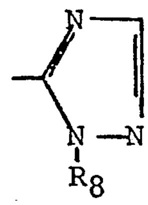 , 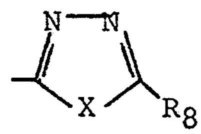 ,

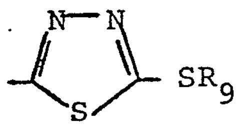 ,

wobei

X = O, S, $NR_8$,

$R_8$= Wasserstoff, Methyl, Äthyl, Isopropyl,

$R_9$= Methyl, Äthyl, Isopropyl,

m = O, 1,

q = O

bedeuten.

Es ist bekannt, zur Bekämpfung von Fäulniserkrankungen an Pflanzen, welche durch Pilze der Gattung Phytophthora verursacht werden, Fungizide aus der Reihe der Dithiocarbamate, wie zum Beispiel Mancozeb oder Maneb, oder Perchlormethylmerkaptanderivate, wie zum Beispiel Captafol zu verwenden. Mit diesen Wirkstoffen können die oberirdisch, an Blatt- und Stengelwerk angreifenden Phytophthorastämme bekämpft werden, während bodenbürtige Erreger der Gattung Phytophthora und Pythium, welche an den Wurzeln der Pflanzen angreifen, nicht erfaßt werden. Es ist weiter bekannt, daß acylierte Anilinoessig-, bzw. -propionsäurederivate, wie sie in den DE-Offenlegungsschriften 23 50 944, 25 13 730, 25 13 789, 25 15 091, 26 43 445 und 26 43 477 beschrieben sind, neben einer herbiziden Wirksamkeit auch eine fungizide Wirkung gegen Pilze der Familie der Oomyceten besitzen.

Es wurde nun überraschenderweise gefunden, daß die erfindungsgemäßen Verbindungen der Formel I eine hervorragende fungizide Wirkung, insbesondere gegen die der Klasse der Phycomyceten angehörenden Oomyceten, wie zum Beispiel Pythium, Phytophthora, Peronospora, Pseudoperonospora und Plasmopara aufweisen.

Damit können Pilze an den verschiedensten Kulturpflanzen, wie zum Beispiel Mais, Reis, Getreide, Zuckerrüben, Gemüse, Gurkengewächse, Kartoffeln, Tomaten, Reben, Hopfen, Tabak, Zitrus- u. Paprikaarten, Zierpflanzen, Kakao, Bananen und Kautschuk bekämpft bzw. gehemmt bzw. ihr Auftreten an diesen Pflanzen ganz verhindert werden. Die Verbindungen der Formel I wirken teilweise systemisch. Sie lassen sich auch als Beizmittel zur Bekämpfung von samenbürtigen Pilzen an Saatgut oder zur Bekämpfung der im Erdboden auftretenden phytopathogenen Pilze einsetzen.

Die Wirkung der erfindungsgemäßen Verbindungen der Formel I ist u.a. deshalb als überaus überraschend zu bezeichnen, weil entsprechende Alkylthioacetanilide, wie sie in der oben genannten DE-OS 25 15 091 beschrieben sind, gegen Phytophthora, insbesondere gegen bodenbürtige Phytophthoraarten und Pilze der Familie Pythium nur eine sehr geringe fungizide Wirkung aufweisen.
Gegenstand der Erfindung sind daher auch fungizide Mittel, die gekennzeichnet sind durch einen Gehalt an einer Verbindung der Formel I.

Die Herstellung von Verbindungen der Formel I mit q = O ist nach verschiedenen Methoden möglich und kann zum Beispiel nach einem der nachstehend aufgeführten Verfahren 1 bis 3 erfolgen:

1. Durch Alkylierung von merkaptosubstituierten Heterocyclen der allgemeinen Formel III, in der Het die Bedeutung wie in Formel I hat, mit Verbindungen der allgemeinen Formel II,

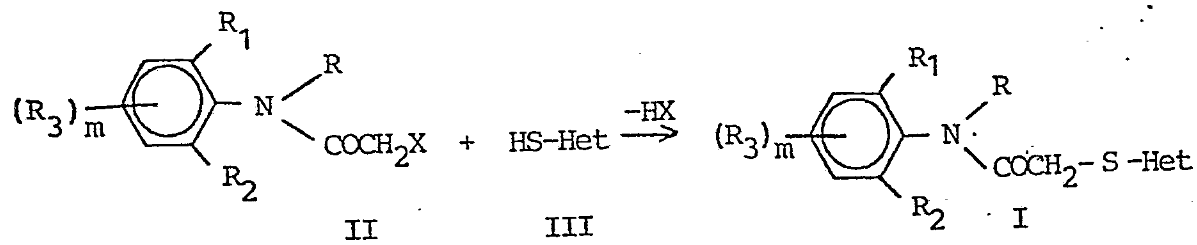


II III I

worin R, $R_1$, $R_2$, $R_3$ und m die Bedeutungen wie in Formel I haben und X eine nukleophil substituierbare Abgangsgruppe, vorzugsweise aus der Gruppe Halogen, Tosylat oder Mesylat, insbesondere Chlor oder Brom, bedeutet.

Die Umsetzung nach dem Verfahren 1 wird bevorzugt in gegenüber den Reaktanten inerten, vorzugsweise polaren oder dipolaren aprotischen Lösungs- oder Verdünnungsmitteln durchgeführt. Als solche kommen zum Beispiel in Frage: Wasser, Alkohole wie Methanol, Ethanol, Propanol, Butanol oder Glykol; dialkylierte Amide wie Dimethylformamid; Nitrile wie Acetonitril oder Propionitril; Ketone wie Aceton, Methylethylketon oder Diisopropylketon; Ether wie Dioxan.

Die Reaktionstemperatur beträgt bevorzugt zwischen 20°C und 160°C, insbesondere zwischen 20°C und 120°C.

Die Umsetzung kann mit oder ohne Zusatz einer Hilfsbase als säurebindendes Mittel erfolgen. Vorzugsweise wird jedoch eine Hilfsbase verwendet. Als solche kommen zum Beispiel in Frage: anorganische Oxide wie Calciumoxid, Hydroxide wie Natriumhydroxid oder Calciumhydroxid, Karbonate wie Natrium- oder Kaliumkarbonat und Trialkylamine wie Triäthylamin oder Pyridin.

2. Durch Umsetzung von Thioglykolsäureaniliden der allgemeinen Formel IV, in der R, $R_1$, $R_2$, $R_3$ und m die Bedeutungen wie in Formel I haben, mit Heterocyclen der allgemeinen Formel V,

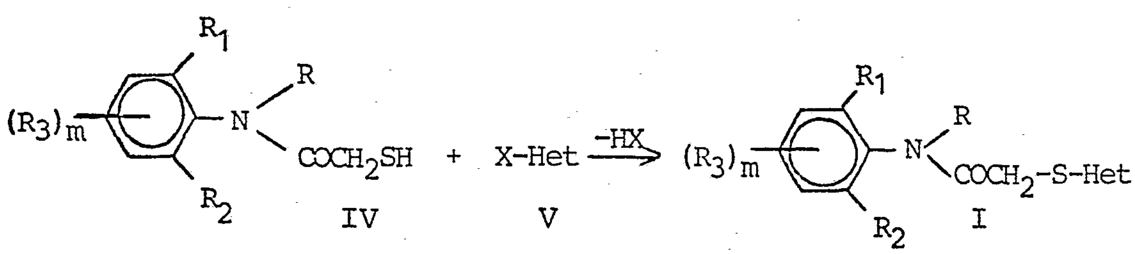


IV + V → I

worin Het die Bedeutung wie in Formel I hat und X eine nukleophil substituierbare Abgangsgruppe, vorzugsweise

aus der Gruppe Halogen, Tosylat oder Mesylat, insbesondere Chlor oder Brom, bedeutet.

Die Reaktionsbedingungen für das Verfahren 2 sind bezüglich der Reaktionstemperatur, dem Reaktionsmedium wie der Verwendung von Hilfsbasen als säurebindende Mittel mit den unter 1. genannten Bedingungen identisch.

3. Durch Umsetzung von Thioglykolsäurechloriden der allgemeinen Formel VI, in der Het die Bedeutung wie in Formel I hat, mit Anilinderivaten der allgemeinen Formel VII,

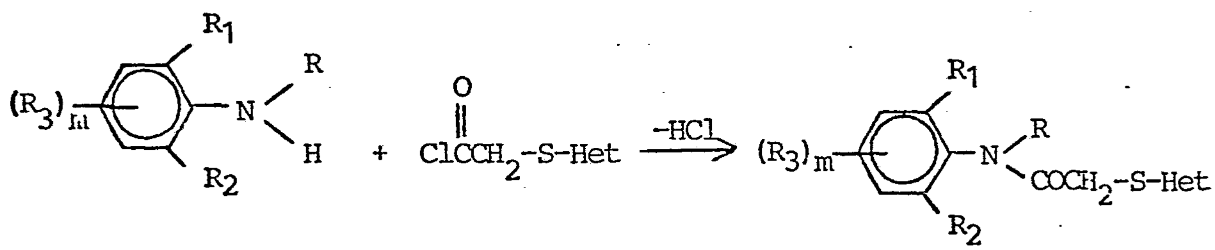


VII VI I

worin R, $R_1$, $R_2$, $R_3$ und m die Bedeutungen wie in Formel I haben, in An- oder Abwesenheit von säurebindenden Mitteln.

Die Umsetzung nach 3. wird bevorzugt in inerten Lösungs- oder Verdünnungsmitteln durchgeführt. Man verwendet zum Beispiel vorzugsweise Kohlenwasserstoffe wie Benzol, Toluol, Xylol, Petrolether, Cyclohexan; halogenierte Kohlenwasserstoffe wie Chlorbenzol, Tetrachlorkohlenstoff; Ether wie Dialkylether, Dioxan oder Tetrahydrofuran; Nitrile wie Acetonitril; Carbonsäureester wie Essigsäureäthylester.

Als säurebindende Mittel kommen zum Beispiel in Frage: Trialkylamine wie Triethylamin, Pyridin, anorganische Basen wie Metalloxide oder -hydroxide, zum Beispiel Calziumoxid oder Natriumhydroxid, oder Carbonate wie Kaliumcarbonat. Wird die Umsetzung ohne säurebindende Mittel durchgeführt, so wird vorteilhaft bei erhöhter Temperatur gearbeitet und es muß für die Abführung des Halogenwasserstoffes aus dem Reaktionsgemisch Sorge getragen werden.

Die Reaktionstemperatur liegt zwischen 0°C und 140°C, bei Anwendung von Hilfsbasen als säurebindende Mittel vorzugsweise zwischen 0°C und 60°C, ohne Hilfsbase zwischen 40°C und 120°C.

Die Herstellung der als Ausgangsmittel verwendeten Anilinderivate der Formel VII kann zum Beispiel erfolgen nach: J. Org. Chem. 30, 4101 (1965); DE-OS 22 12 268; US-PS 40 12 519; Tetrahedron 1967; 487 und 493. Aus diesen Anilinderivaten lassen sich auch die Ausgangsmaterialien der Formel II durch Acylierung, zum Beispiel mit Chloracetylchlorid oder Bromacetylchlorid gewinnen.

Die Herstellung von Verbindungen der Formel I mit q = 1 oder 2 erfolgt durch Oxidation von Verbindungen der Formel I mit q = 0 nach folgendem Reaktionsschema:

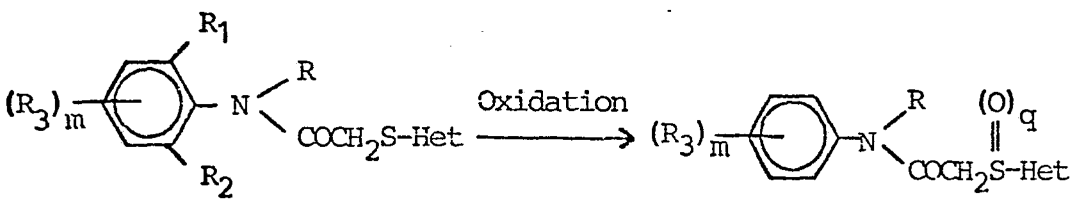


I (q=0) I (q=1 oder 2)

Die Oxidation von Verbindungen der Formel I mit q = 0 unter Aufnahme von 1 Sauerstoffatom zu Sulfoxiden der Formel I mit q=1 bzw. unter Aufnahme von 2 Sauerstoffatomen zu Sulfonen der Formel I mit q=2 kann nach dem Fachmann geläufigen Methoden durchgeführt werden. Als Oxidationsmittel kommen zum Beispiel in Frage:
Wasserstoffperoxid [Chem. Ber. 41, 2836 (1908); Houben-Weyl Bd. 9, 4. Auflage, S. 228], Persäuren wie Peressigsäure, Perbenzoesäure, 3-Chlorperbenzoesäure oder Phthalmonopersäure [Houben-Weyl, Bd. 9, 4. Auflage, S. 228; J. prak. Chem. 131, 357 (1931) und 128, 171 (1930); Chem. Ber. 70, 379 (1937)], Chrom- oder Salpetersäure oder im Falle der Sulfoxide auch Brom oder Chlor [Synth. 1/1979 S. 39].

Die Oxidationsreaktionen werden bevorzugt in Gegenwart von Lösungsmitteln durchgeführt. Als solche können verwendet werden: Kohlenwasserstoffe wie zum Beispiel Benzol, halogenierte Kohlenwasserstoffe wie Methylenchlorid, Chloroform, Tetrachlorkohlenstoff, Dichlorethan oder Trichlorethylen, Ketone wie Aceton oder Methylethylketon, Carbonsäuren wie Eisessig oder Propionsäure, Anhydride wie Acetanhydrid und Wasser oder auch Gemische dieser Lösungsmittel untereinander.

Die Reaktionstemperaturen liegen zur Herstellung von Sulfonen zwischen -20°C und 160°C, vorzugsweise zwischen 0°C und 120°C, zur Herstellung von Sulfoxiden zwischen -40°C und 60°C.

Wird die Herstellung erfindungsgemäßer Verbindungen der Formel I nach einem der vorstehend beschriebenen Verfahren 1 oder 2 in solchen Lösungsmitteln durchgeführt, in denen einer der Reaktanten nur wenig löslich ist, so kann eine Beschleunigung der Umsetzung durch die Zugabe von Katalysatoren, wie zum Beispiel Kronenethern oder die als Phasentransferkatalysatoren bekannten quartären Ammonium-, bzw. Phosphoniumsalze erreicht werden.

Die Verbindungen der Formel I mit R = 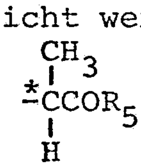 und 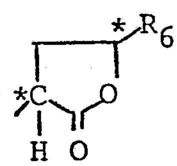

besitzen asymmetrische Kohlenstoffatome. Sie liegen daher im allgemeinen als racemische Gemische enantiomerer Formen vor. Die enantiomeren Formen weisen eine unterschiedliche biologische Aktivität auf. Eine gezielte Synthese des reinen D-Isomeren kann zum Beispiel durch fraktionierte Kristallisation von Salzen der Anilinopropionsäure der allgemeinen Formel VIII mit optisch aktiven Basen

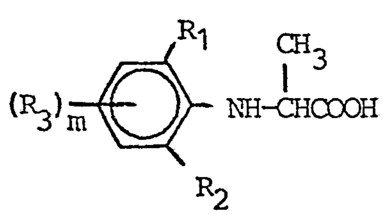

(VIII)

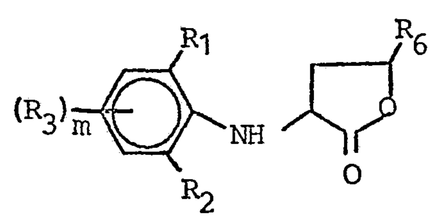

(VIIIa)

wie Cinchonin oder α-Phenylethylamin oder durch fraktionierte Kristallisation von Anilinobutyrolactonen der allgemeinen Formel VIIIa mit optisch aktiven Säuren, wie zum Beispiel D- oder L-Weinsäure erfolgen. In den Formeln VIII und VIIIa haben $R_1$, $R_2$, $R_3$, $R_6$ und m die Bedeutungen wie in Formel I.

Es ist aber auch eine gezielte Synthese, ausgehend von optisch aktiven Halogenpropionsäurederivaten, wie zum Beispiel L-2-Chlorpropionsäuremethylester, optisch aktiven Milchsäureestertosylaten oder optisch aktiven Halogenbutyrolactonen und Anilinen der allgemeinen Formel IX,

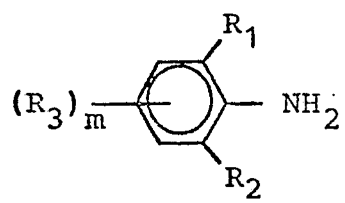

(IX)

in der $R_1$, $R_2$, $R_3$ und m die Bedeutungen wie in Formel I haben, möglich. Diese Umsetzung erfolgt vorwiegend als SN2-Reaktion unter Inversion der Konfiguration. So entstehen aus 2,6-Dimethylanilin und L-2-Chlorpropionsäure bzw. L-2-Chlorpropionsäuremethylester bevorzugt D-2-(2,6-Dimethylanilino)-propionsäure bzw. deren Methylester.

Die erfindungsgemäßen Mittel enthalten die Wirkstoffe der Formel I im allgemeinen zu etwa 2 - 95 Gew.-%, vorzugsweise 5 - 90 Gew.-%. Sie können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, zum Beispiel polyoxethylierte Alkylphenole, polyoxethylierte Fettalkohole, Alkyl- oder Alkylphenyl-sulfonate und Dispergiermittel, zum Beispiel ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise, zum Beispiel durch Mahlen und Vermischen der Komponenten. Emulgierbare Konzentrate können zum Beispiel durch Auflösen des Wirkstoffes in einem inerten organischen Lösungsmittel, zum Beispiel Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgaoren können beispielsweise verwendet werden: Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel kann man durch Vermahlen des Wirkstoffes mit fein verteilten, festen Stoffen, zum Beispiel Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, zum Beispiel Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - granuliert werden.

In Spritzpulvern beträgt die Wirkstoffkonzentration zum Beispiel etwa 10 - 90 Gew.-%, der Rest zu 100 Gew.-% besteht aus üblichen Formulierungsbestandteilen. Bei emulgierbaren Konzentraten kann die Wirkstoffkonzentration etwa 10 - 80 Gew.-% betragen. Staubförmige Formulierungen enthalten meistens 5 - 20 Gew.-% an Wirkstoff, versprühbare Lösungen etwa 2 - 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel-, Füll- oder Trägerstoffe.

Zur Anwendung werden die in handelsüblicher Form vorliegenden Konzentrate gegebenenfalls in üblicher Weise verdünnt, zum Beispiel bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen pestiziden Wirkstoffen, wie zum Beispiel Insektiziden, Akariziden, Herbiziden, Wachstumsregulatoren oder Fungiziden sind gegebenenfalls möglich. Insbesondere bei Mischungen mit Fungiziden können teilweise auch synergistische Wirkungssteigerungen erzielt werden.

Die Erfindung wird durch die folgenden Beispiele näher erläutert:

## A. Herstellungsbeispiele

Beispiel 1

Methyl-2-/N-thiazolin-2-ylmerkaptoacetyl)-N-(2,6-dimethylphenyl)-amino7-propanoat.

17 g (0,06 Mol) Methyl-2-/N-(chloracetyl)-N-(2,6-dimethylphenyl)-amino7-propanoat, 6,3 g (0,063 Mol) 2-Merkaptothiazolin und 0,7 g (0,063 Mol) Kaliumkarbonat werden in 100 ml Acetonitril 16 Stunden bei 65°C gerührt. Nach Abfiltrieren des entstandenen anorganischen Salzes wird das Filtrat mit Eis und Wasser verdünnt, der gebildete Niederschlag abfiltriert und aus Toluol umkristallisiert. Man erhält 18,4 g (84 % d.Th.) Methyl-2-/N-(thiazolin-2-ylmerkaptoacetyl)-N-(2,6-dimethylphenyl)-amino7-propanoat mit dem Schmelzpunkt von 95°C.

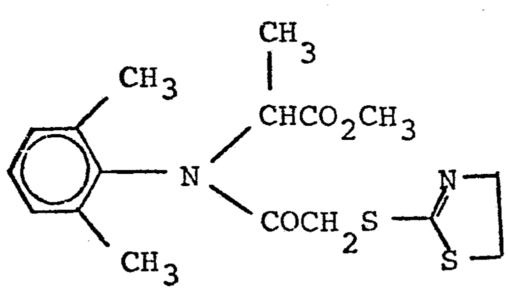

Beispiele 2 bis 28a

In analoger Weise wie in Beispiel 1 beschrieben werden die nachstehend in der Tabelle 1 aufgeführten Beispiele 2 bis 28a hergestellt. Es sind in der Tabelle 1 für die in den Beispielen 2 bis 28a hergestellten Verbindungen der Formel I mit q = O die Reste R, $R_1$ bis $R_3$, m und Het in zusammengefaßter Form wiedergegeben und in der letzten Tabellenspalte die ermittelten physikalisch-chemischen Kenndaten aufgeführt.

Tabelle 1

Formel I, q = O:

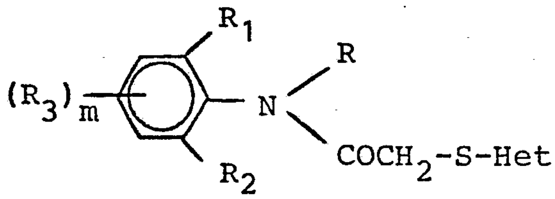

| Bsp. Nr. | $R_1$ | $R_2$ | $R_3$ | m | R | Het | physikal.-chem. Kenndaten |
|---|---|---|---|---|---|---|---|
| 2 | $-CH_3$ | $-CH_3$ | – | 0 | $CH_3$<br>$-CHCO_2CH_3$ | 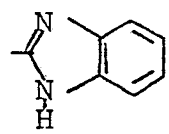 | Fp. 110 - 113°C |
| 3 | $-CH_3$ | $-CH_3$ | – | 0 | $CH_3$<br>$-CHCO_2CH_3$ | 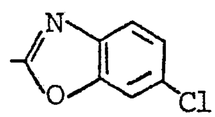 | Fp. 107 - 110°C |
| 4 | $-CH_3$ | $-CH_3$ | – | 0 | $CH_3$<br>$-CHCO_2CH_3$ | 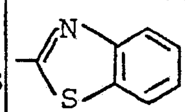 | Fp. 99 - 101°C |
| 5 | $-CH_3$ | $-CH_3$ | – | 0 | $CH_3$<br>$-CHCO_2CH_3$ | 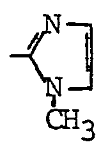 | Fp. 80 - 81°C |
| 6 | $-CH_3$ | $-CH_3$ | – | 0 | $CH_3$<br>$-CHCO_2CH_3$ | 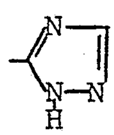 | Fp. 140 - 142°C |
| 7 | $-CH_3$ | $-CH_3$ | – | 0 | $CH_3$<br>$-CHCO_2CH_3$ | 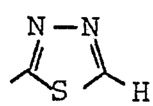 | Fp. 95 - 96°C |
| 8 | $-CH_3$ | $-CH_3$ | – | 0 | $CH_3$<br>$-CHCO_2CH_3$ | 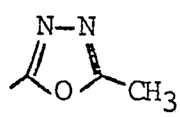 | Fp. 110 - 118°C |
| 9 | $-CH_3$ | $-CH_3$ | – | 0 | $CH_3$<br>$-CHCO_2CH_3$ | 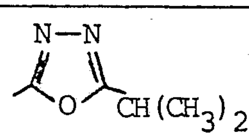 | Fp. 106 - 107°C |
| 10 | $-CH_3$ | $-CH_3$ | – | 0 | $CH_3$<br>$-CHCO_2CH_3$ | 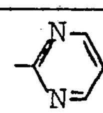 | $n_D^{23}$ = 1,5703 |

(Tabelle 1, Fortsetzung)

| Bsp. Nr. | $R_1$ | $R_2$ | $R_3$ | m | R | Het | physikal.-chem. Kenndaten |
|---|---|---|---|---|---|---|---|
| 11 | $-CH_3$ | $-CH_3$ | - | 0 | 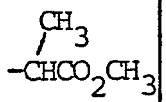 | 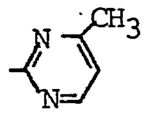 | $n_D^{23}$ = 1,5753 |
| 12 | $-CH_3$ | $-CH_3$ | - | 0 | 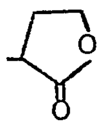 | 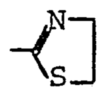 | Fp. 161 - 162°C |
| 13 | $-CH_3$ | $-CH_3$ | - | 0 | 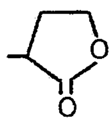 | 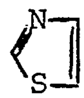 | |
| 14 | $-CH_3$ | $-CH_3$ | - | 0 | 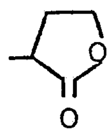 | 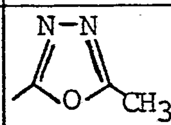 | |
| 15 | $-CH_3$ | $-CH_3$ | - | 0 | 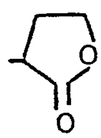 | 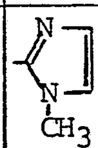 | |
| 16 | $-CH_3$ | $-CH_3$ | - | 0 | 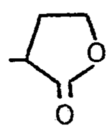 | 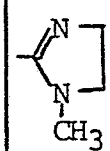 | |
| 17 | $-CH_3$ | $-CH_3$ | - | 0 | 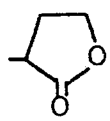 | 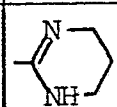 | |
| 18 | $-CH_3$ | $-CH_3$ | - | 0 | 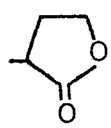 | 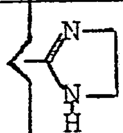 | |
| 19 | $-CH_3$ | $-CH_3$ | - | 0 | 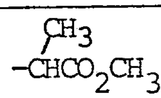 | 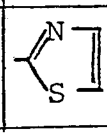 | |

(Tabelle 1, Fortsetzung)

| Bsp. Nr. | $R_1$ | $R_2$ | $R_3$ | m | R | Het | physikal.-chem. Kenndaten |
|---|---|---|---|---|---|---|---|
| 20 | $-CH_3$ | $-CH_3$ | - | 0 | $CH_3$<br>$-CHCO_2CH_3$ | 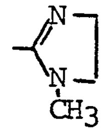 | |
| 21 | $-CH_3$ | $-CH_3$ | - | 0 | $CH_3$<br>$-CHCO_2CH_3$ | 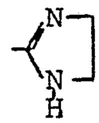 | |
| 22 | $-CH_3$ | $-CH_3$ | - | 0 | $CH_3$<br>$-CHCO_2CH_3$ | 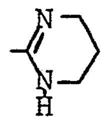 | |
| 23 | $-CH_3$ | $-C_2H_5$ | - | 0 | $CH_3$<br>$-CHCO_2CH_3$ | 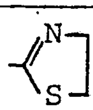 | |
| 24 | $-CH_3$ | -Cl | - | 0 | $CH_3$<br>$-CHCO_2CH_3$ | 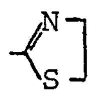 | |
| 25 | $-CH_3$ | -Cl | - | 0 | $CH_3$<br>$-CHCO_2CH_3$ | 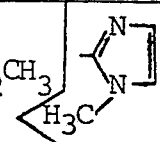 | |
| 26 | $-CH_3$ | -Cl | - | 0 | $CH_3$<br>$-CHCO_2CH_3$ | 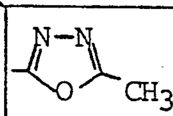 | |
| 27 | $-CH_3$ | -Cl | - | 0 | $CH_3$<br>$-CHCO_2CH_3$ | 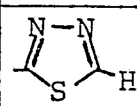 | |
| 28 | $-CH_3$ | $-CH_3$ | - | 0 | $CH_3$<br>$-CHCO_2CH_3$ | 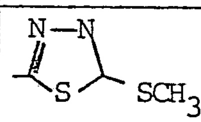 | |
| 28a | $-CH_3$ | $-CH_3$ | - | 0 | $CH_3$<br>$-CHCO_2CH_3$ | 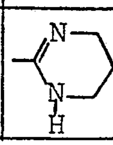 | Fp. 171-172,5°C |

Beispiel 29

Methyl-2-/N̄-(benzthiazol-2-ylsulfonylacetyl)-N-(2,6-dimethylphenyl)amino/-propanoat.

14,50 g (0,035 Mol) Methyl-2-/N̄-(benzthiazol-2-ylmerkaptoacetyl)-N-(2,6-dimethylphenyl)-amino/-propionat, gelöst in 100 ml Methylenchlorid werden bei 30°C bis 40°C mit 0,75 Mol (15,25 g) 85 %iger 3-Chlorperbenzoesäure versetzt. Nach Zugabe wird 1 Stunde bei Rückfluß nachgerührt, anschließend 3-Chlorbenzoesäure abfiltriert und das Filtrat mit Natriumbikarbonatlösung gewaschen. Die Methylenchloridphase wird getrocknet und Methylenchlorid abdestilliert. Der verbleibende Rückstand wird aus Toluol/Cyclohexan umkristallisiert. Man erhält 14,2 g (90,5 % d.Th.) Methyl-2-/N̄-(benzthiazol-2-ylsulfonylacetyl)-N-(2,6-dimethylphenyl)-amino/-propanoat mit dem Schmelzpunkt 181°C bis 182,5°C.

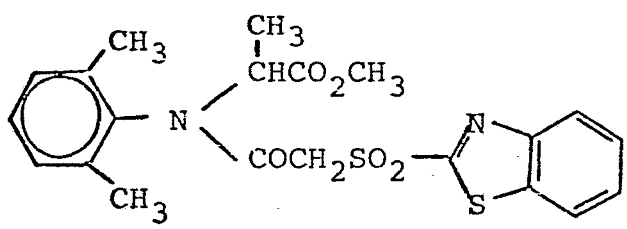

Beispiel 30

Methyl-2-/N̄-(benzthiazol-2-ylsulfinylacetyl)-N-(2,6-dimethylphenyl)-amino/-propanoat.

10,7 g (0,025 Mol) Methyl-2-/N̄-(benzthiazol-2-ylmerkaptoacetyl)-N-(2,6-dimethylphenyl)-amino/-propanoat, gelöst in 100 ml Methylenchlorid werden bei -20°C im Verlauf einer Stunde mit 5,1 g (0,025 Mol) 85 %iger 3-Chlorperbenzoesäure, gelöst in 50 ml Methylenchlorid, versetzt. Nach Zugabe wird 2 Stunden bei Raumtemperatur nachgerührt, die Methylenchloridphase gründlich mit wäßriger Natriumhydrogenkarbonatlösung gewaschen, über Natriumsulfat getrocknet, schließlich Methylenchlorid abdestilliert und der verbleibende Rückstand aus Toluol/Cyclohexan umkristallisiert. Man erhält 8,4 g (78 % d.Th.) Methyl-2-

/N̄-(benzthiazol-2-ylsulfinylacetyl)-N-(2,6-dimethylphenyl)-amino/-propanoat mit dem Schmelzpunkt 159°C bis 161°C.

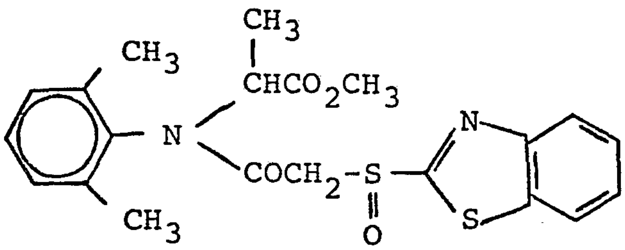

B. Formulierungsbeispiele

Beispiel A

Ein Stäubemittel wird erhalten, indem man 10 Gewichtsteile Wirkstoff und 90 Gewichtsteile Talkum als Inertstoff mischt und in einer Schlagmühle zerkleinert.

Beispiel B

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Calcium und 1 Gewichtsteil oleoylmethyltaurinsaures Natrium als Netz- und Dispergiermittel mischt und in einer Stiftmühle mahlt.

Beispiel C

Ein in Wasser leicht dispergierbares Dispersionskonzentrat wird erhalten, indem man 20 Gewichtsteile Wirkstoff mit 6 Gewichtsteilen Nonylphen-olpolyglykolether (10 AeO)*), 3 Gewichtsteilen Isotridecanolpolyglykolether (8 AeO)*) und 71 Gewichtsteilen paraffinischem Mineralöl (Siedebereich zum Beispiel ca. 255 bis über 377°C) mischt und in einer Reibkugelmühle auf eine Feinheit von unter 5 Mikron vermahlt.

*) = Anzahl Ethylenoxydeinheiten im Polyglykoletherrest

Beispiel D

Ein emulgierbares Konzentrat wird erhalten aus 15 Gewichtsteilen Wirkstoff, 75 Gewichtsteilen Cyclohexanon als Lösungsmittel und 10 Gewichtsteilen Nonylphenolpolyglykolether (10 AeO) als Emulgator.

Beispiel E

Zur Herstellung eines 5 % Wirkstoff enthaltenden Granulates werden die folgenden Komponenten verwendet: 5 Gew.-Teile Wirkstoff, 0,25 Gew.-Teile Epichlorhydrin, o,25 Gew.-Teile Cetylpolyglykolether, 3,5 Gew.-Teile Polyethylenglykol, 91 Gew.-Teile Kaolin. Es werden der Wirkstoff mit dem Epichlorhydrin vermischt und in 6 Gew.-Teilen Aceton gelöst, dann das Polyethylenglykol und der Cetylether zugesetzt. Die resultierende Lösung wird auf Kaolin aufgesprüht und anschließend das Aceton unter Vakuum verdampft.

C. Biologische Beispiele

Beispiel I

Wirkung der Verbindungen der Formel I gegen Pythium ultimum an Erbsen

a) Wirkung nach Bodenapplikation

Der Pilz wird in einer Sandkultur angezogen und mit Erde vermischt. Die so infizierte Erde wird in Anzuchttöpfe abgefüllt und mit Erbsensaatgut besät. Nach der Aussaat werden die zu prüfenden fungiziden Verbindungen als wäßrige Suspensionen jeweils auf die Oberfläche des Kulturbodens gegossen. Dabei werden Wirkstoffkonzentrationen von 200 bis 12 ppm (bezogen auf das Bodenvolumen) angewandt. Die Töpfe werden 2 - 3 Wochen in einem Gewächshaus mit 20°C bis 22°C aufgestellt und der Kulturboden gleichmäßig leicht feucht gehalten. Bei der Auswertung auf Pythium-Befall werden der Auflauf der Erbsenpflanzen sowie der Anteil gesunder und kranker

Pflanzen im Vergleich zu den entsprechenden Kontrollen ermittelt und entsprechend der Wirkungsgrad errechnet.

Die beanspruchten Verbindungen der Formel I führen bei einer Aufwandmenge von 200 ppm zu einer weitgehenden Kontrolle des Pythium ultimum-Befalls (Wirkungsgrad durchschnittlich $>$80 %). Eine Reihe von Verbindungen der Formel I ist bei noch wesentlich geringerer Wirkstoffkonzentration ausgezeichnet wirksam, wie die in der nachfolgenden Tabelle I zusammengefaßten Versuchsergebnisse zeigen.

Tabelle I

Wirkung gegen Pythium ultimum nach Behandlung von beimpfter Erde und anschließender Aussaat von gesundem Saatgut.

1. Tag: Beimpfung des Bodens mit Pythium ultimum und Behandlung mit fungizider Verbindung, Aussaat

14. Tag: Auswertung

| Verbindung gem. Beispiel Nr. | Wirkungsgrad in % bei ppm Wirkstoff im Boden | | |
|---|---|---|---|
| | 200 ppm | 100 ppm | 50 ppm |
| 1 | 100 | 100 | 100 |
| 2 | 100 | - | - |
| 5 | 100 | 100 | 100 |
| 7 | 100 | 100 | - |
| 8 | 100 | - | - |
| 10 | 100 | 100 | 100 |
| Vergleichsmittel A* | 65 | 40 | 0 |

| Verbindung gem. Beispiel Nr. | Wirkungsgrad in % bei ppm Wirkstoff im Boden 200 ppm | 100 ppm | 50 ppm |
|---|---|---|---|
| Kontrolle: unbehandelte, beimpfte Erde | 0 (= 100 % Auflaufschäden) | | |
| unbehandelte, nicht beimpfte Erde | 100 % Auflauf | | |

*) Vergleichsmittel A: Methyl-2-/N-(methylthioacetyl)-N-(2,6-dimethylphenyl)-amino/-propanoat (vgl. DE-OS 25 15 091).

Beispiel II

Präventive Wirkung der Verbindungen der Formel I gegen Phytophthora capsici (Bodenapplikation)

Phytophthora capsici wird in Sandkulturen angezogen und mit Versuchserde vermischt. Der so infizierte Boden wird in Anzuchttöpfe abgefüllt und mit Paprika (Capsicum annuum) besät. Nach der Aussaat werden die beanspruchten Verbindungen als wäßrige Suspensionen auf die Bodenoberfläche gegossen oder in die Abdeckerde eingearbeitet. Dabei werden Wirkstoffkonzentrationen von 200 bis 12 ppm (bezogen auf das Bodenvolumen) angewandt. Die Töpfe werden anschließend ca. 3 Wochen in ein Gewächshaus bei 22°C bis 24°C gestellt und der Kulturboden gleichmäßig feucht gehalten. Bei der Auswertung auf Phytophthora capsici-Befall werden der Auflauf der Paprika-Pflanzen sowie der Anteil gesunder und kranker Pflanzen im Vergleich zu den entsprechenden Kontrollen ermittelt und entsprechend der Wirkungsgrad errechnet.

Die Verbindungen der Formel I ergeben bei einer Aufwandmenge von 200 ppm eine nahezu vollständige Kontrolle des

Phytophthora capsici-Befalls. Eine Reihe von Verbindungen der Formel I führt bei noch wesentlich geringerer Aufwandmenge zu einer ebenfalls nahezu vollständigen Reduktion des Pilzbefalls, wie nachfolgend die in der Tabelle II zusammengefaßten Versuchsergebnisse zeigen.

Tabelle II

Wirkung gegen Phytophthora capsici nach Behandlung von beimpfter Erde mit fungiziden Verbindungen der Formel I und Aussaat von gesundem Paprika-Saatgut.

1. Tag: Beimpfung des Bodens und Behandlung mit fungizider Verbindung, Aussaat

ca. 21. Tag: Auswertung

| Verbindung gem. Beispiel Nr. | Wirkungsgrad in % bei ppm Wirkstoff im Boden | | |
|---|---|---|---|
| | 200 ppm | 100 ppm | 50 ppm |
| 1 | 100 | 100 | 100 |
| 3 | 100 | 100 | - |
| 2 | 100 | 100 | - |
| 5 | 100 | 100 | 100 |
| 8 | 100 | 100 | - |
| 10 | 100 | 100 | 100 |
| Kontrolle: | | | |
| unbehandelte, beimpfte Erde | 0 (= 100 % Auflaufschäden) | | |
| unbehandelte, nicht beimpfte Erde | 100 % Auflauf | | |

Beispiel III

Wirkung der Verbindungen der Formel I gegen Phytophthora infestans auf Solanum lycopersicum

Präventive Wirkung

Tomatenpflanzen (Solanum lycopersicum) der Sorte Rheinlandsruhm werden im 3-Blattstadium mit den beanspruchten Verbindungen der Formel I tropfnaß gespritzt. Die Anwendungskonzentrationen betragen jeweils 500, 250 und 125 mg Wirkstoff pro Liter Spritzbrühe.

Nach dem Antrocknen des Spritzbelages werden die Pflanzen mit einer Zoosporangiensuspension von Phytophthora infestans stark inokuliert und einen Tag lang tropfnaß in eine Klimakammer mit einer Temperatur von 16°C und einer relativen Luftfeuchte von fast 100 % gestellt. Anschließend werden sie in ein Kühlgewächshaus mit einer Temperatur von 15°C und einer relativen Luftfeuchtigkeit von 90 - 95 % gestellt. Nach einer Inkubationszeit von 7 Tagen werden die Pflanzen auf Befall mit Phytophthora untersucht. Letzterer wird in Form von typischen Blattflecken sichtbar, deren Anzahl und Größe als Bewertungsmaßstab für die geprüften Wirkstoffe dienen. Der Befallsgrad wird ausgedrückt in % befallener Blattfläche im Vergleich zu unbehandelten, infizierten Kontrollpflanzen (= 100 % Befall). Bei diesem Test bewirken die Verbindungen der Formel I bei einer Aufwandmenge von 500 mg/Ltr. Spritzbrühe eine nahezu vollständige Befallsverhinderung. An einigen in der Tabelle III zusammengefaßten Versuchsergebnissen kann gezeigt werden, daß viele Verbindungen der Formel I auch bei wesentlich geringeren Aufwandmengen zu einer vollständigen Pilzbefallskontrolle führen.

Tabelle III

| Verbindung gem. Beispiel Nr. | % Phytophthora-Befall bei mg Wirkstoff/Ltr. Spritzbrühe 500 mg | 250 mg | 125 mg |
|---|---|---|---|
| 1 | 0 | - | - |
| 8 | 0 | 0 | 0 |
| 10 | 0 | 0 | 0 |
| 11 | 0 | - | - |

| Verbindung gem. Beispiel Nr. | % Phytophthora-Befall bei mg Wirkstoff/ Ltr. Spritzbrühe 500 mg | 250 mg | 125 mg |
| --- | --- | --- | --- |
| unbehandelte, infizierte Pflanzen | 100 | | |

Beispiel IV

Wirkung der Verbindungen der Formel I gegen Plasmopara viticola auf Reben

Präventive Wirkung

Weinpflanzen, die aus Stecklingen der Plasmopara-anfälligen Sorte Müller-Thurgau gezogen waren, werden im 4-Blattstadium mit wäßrigen Suspensionen der zu prüfenden Verbindungen der Formel I tropfnaß gespritzt. Die Anwendungskonzentrationen betragen 500, 250 und 125 mg Wirkstoff/l Spritzbrühe. Nach dem Antrocknen des Spritzbelages werden die Pflanzen mit einer Zoosporangiensuspension von Plasmopara viticola inokuliert und tropfnaß in eine Klimakammer mit einer Temperatur von 20°C und einer relativen Luftfeuchte von ca. 100 % gestellt. Nach 24 Stunden werden die infizierten Pflanzen der Klimakammer entnommen und in ein Gewächshaus mit einer Temperatur von 23°C und einer Luftfeuchtigkeit von 98 % gebracht. Nach einer Inkubationszeit von 7 Tagen werden die Pflanzen angefeuchtet, über Nacht in die Klimakammer gestellt und die Krankheit zum Ausbruch gebracht. Anschließend erfolgt die Befallsauswertung. Anzahl und Größe der Infektionsstellen auf den Blättern der inokulierten und behandelten Pflanzen dienen als Maßstab für die Wirksamkeit der beanspruchten Verbindungen. Der Befallsgrad wird ausgedrückt in % befallener Blattfläche im Vergleich zu unbehandelten, infizierten Kontrollpflanzen (= 100 % Befall).

Die Verbindungen der Formel I geben bei einer Aufwandmenge von 500 mg Wirkstoff/Ltr. Spritzbrühe eine durchschnittliche Befallskontrolle von > 80 %. Wie an einigen in der Tabelle IV zusammengefaßten Versuchsergebnissen gezeigt werden kann, können viele Verbindungen der Formel I auch bei wesentlich geringeren Aufwandmengen zu einer vollständigen Pilzbefallskontrolle führen.

Tabelle IV

| Verbindung gem. Beispiel Nr. | % Plasmopara-Befall bei mg Wirkstoff/l Spritzbrühe 500 mg | 250 mg | 125 mg |
|---|---|---|---|
| 1 | 0 | 0 | 0 |
| 2 | 0 | - | - |
| 5 | 0 | 0 | 0 |
| 7 | 0 | 0 | 0 |
| 8 | 0 | 0 | 0 |
| 9 | 0 | - | - |
| 10 | 0 | 0 | 0 |
| unbehandelte, infizierte Pflanzen | 100 | | |

0040310

HOE 80/F 071

Patentansprüche

1. Verbindungen der allgemeinen Formel I,

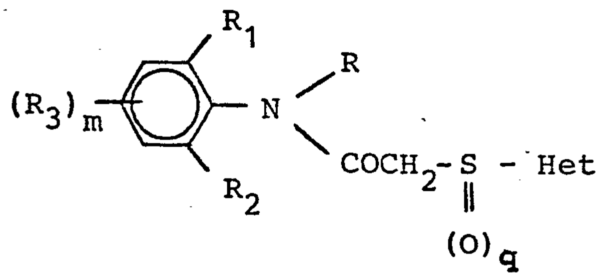

(I)

worin

R = 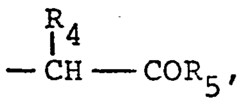, 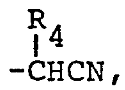, 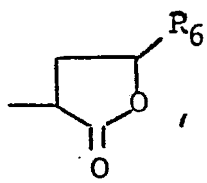,

$R_1$ = $(C_1 - C_3)$-Alkyl, Halogen, $(C_1 - C_3)$-Alkoxy, $(C_1 - C_2)$-Alkylthio, $(C_2 - C_4)$-Alkenyl,

$R_2$ = Wasserstoff, $(C_1 - C_2)$-Alkyl,

$R_3$ = Methyl, Halogen,

$R_4$, $R_6$ = Wasserstoff, Methyl,

$R_5$ = Hydroxy, $(C_1 - C_4)$-Alkoxy, $(C_1 - C_3)$-Alkylthio, $(C_1 - C_2)$-Alkoxyäthoxy, Amino, $(C_1 - C_2)$-Alkylamino, Di-$(C_1 - C_2)$-alkylamino, O-Kat, wobei Kat für ein Kationenäquivalent einer anorganischen oder organischen Base steht, vorzugsweise Na, K, 1/2 Ca, Ammonium,

Het = ein 5 - 6 gliedriger, gesättigter oder ungesättigter Heterocyclus, gegebenenfalls benzokondensiert, mit bis zu 4 Heteroatomen aus der Gruppe N, O, S, vorzugsweise ein Heterocyclus aus der nachfolgend genannten Reihe:

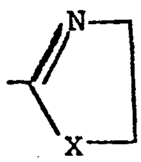, 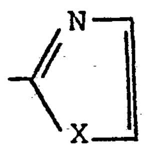, 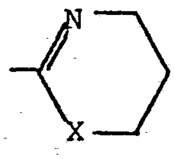, 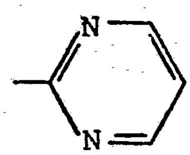,

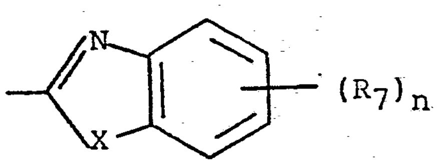, 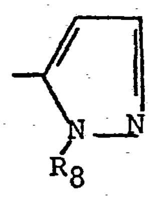, 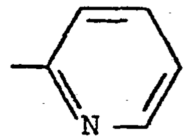,

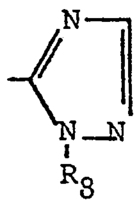, 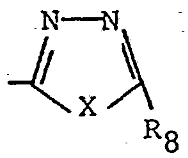, 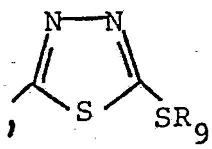, 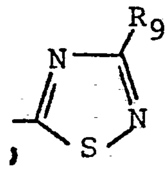,

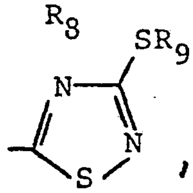,

wobei jeder Heterocyclus zusätzlich im Kohlenstoffteil eine oder mehrere $(C_1-C_4)$-Alkylgruppen tragen kann, und

X = O, S, $NR_8$,

$R_8$= Wasserstoff, $(C_1 - C_4)$-Alkyl,

$R_7$= $(C_1 - C_4)$-Alkyl, Halogen,

$R_9$= $(C_1 - C_4)$-Alkyl,

n = O, 1,

m = O, 1, 2,

q = O, 1, 2

bedeuten.

2. Verfahren zur Herstellung von Verbindungen der Formel I gemäß Anspruch 1, dadurch gekennzeichnet,

I. daß man zur Herstellung von Verbindungen der Formel I, worin q= O bedeutet,

a) Verbindungen der allgemeinen Formel II,

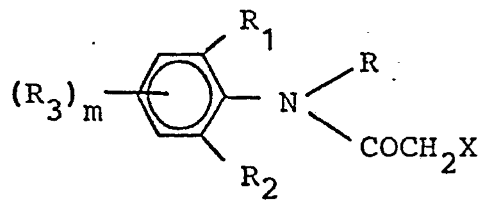

(II)

worin R, $R_1$, $R_2$, $R_3$ und m die Bedeutung wie in Formel I haben und X eine nukleophil substituierbare Abgangsgruppe, vorzugsweise aus der Gruppe Halogen, Tosylat oder Mesylat, insbesondere Chlor oder Brom, bedeutet, mit Heterocyclen der allgemeinen Formel III,

HS-Het (III)

in der Het die Bedeutung wie in Formel I hat, umsetzt, gegebenenfalls unter Zusatz von säurebindenden Mitteln, vorzugsweise in inerten Lösungs- oder Verdünnungsmitteln und bevorzugt bei Temperaturen zwischen 20 und 160°C,

oder

b) daß man Thioglykolsäureanilide der allgemeinen Formel IV,

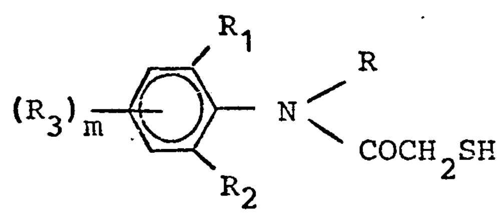

(IV)

in der R, $R_1$, $R_2$, $R_3$ und m die Bedeutungen wie in Formel I haben, mit Heterocyclen der allgemeinen Formel V,

X-Het (V)

worin Het die Bedeutung wie in Formel I hat und X eine nukleophil substituierbare Abgangsgruppe, vorzugsweise aus der Gruppe Halogen, Tosylat oder Mesylat, insbesondere Chlor oder Brom, bedeutet, umsetzt, gegebenenfalls unter Zusatz von säurebindenden Mitteln, vorzugsweise in inerten Lösungs- oder Verdünnungsmitteln und bevorzugt bei Temperaturen zwischen 20 und 160°C,

oder

c) daß man Anilinderivate der allgemeinen Formel VII,

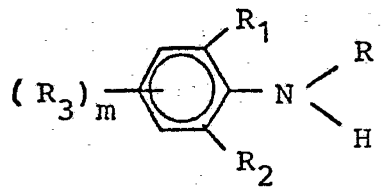

(VII)

worin R, $R_1$, $R_2$, $R_3$ und m die Bedeutungen wie in Formel I haben, mit Thioglykolsäurechloriden der allgemeinen Formel VI,

$$Cl-\overset{O}{\overset{\|}{C}}-CH_2-S-Het \qquad (VI)$$

in der Het die Bedeutung wie in Formel I hat, umsetzt, gegebenenfalls unter Zusatz von säurebindenden Mitteln, vorzugsweise in inerten Lösungs- oder Verdünnungsmitteln und bevorzugt bei Temperaturen zwischen 0 und 140°C,

und

II. daß man zur Herstellung von Verbindungen der Formel I, worin q = 1 oder 2 bedeutet, Verbindungen der Formel I, worin q = 0 bedeutet, mit entsprechenden Mengen eines Oxidationsmittels entweder zu Verbindungen der Formel I, in der q = 1 bedeutet, oder zu Verbindungen der Formel I, in der q = 2 bedeutet, umsetzt, vorzugsweise jeweils in Lösungs- oder Verdünnungsmitteln und bevorzugt bei Temperaturen zwischen -40 und 60°C für Verbindungen der Formel I mit q = 1, bzw. zwischen -20 und 160°C für Verbindungen der Formel I mit q = 2.

3. Fungizide Mittel, gekennzeichnet durch einen Gehalt an einer Verbindung der Formel I gemäß Anspruch 1 als Wirkstoff.

4. Fungizide Mittel, gekennzeichnet durch einen Gehalt von 2 bis 95 Gew.-%, vorzugsweise 5 bis 90 Gew.-% an einer Verbindung der Formel I gemäß Anspruch 1 als Wirkstoff sowie üblichen Formulierungshilfsmitteln.

5. Verwendung von Verbindungen der Formel I gemäß Ansprüchen 1, 3 und 4 zur Schädlingsbekämpfung im Pflanzenschutz.

6. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1, 3 und 4 in Kontakt bringt.

Patentansprüche für Österreich 

1. Verfahren zur Herstellung von Verbindungen der allgemeinen Formel I,

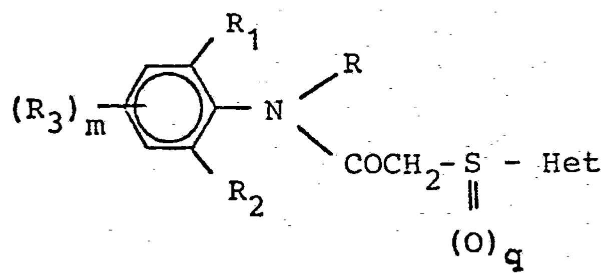

(I)

worin

R = 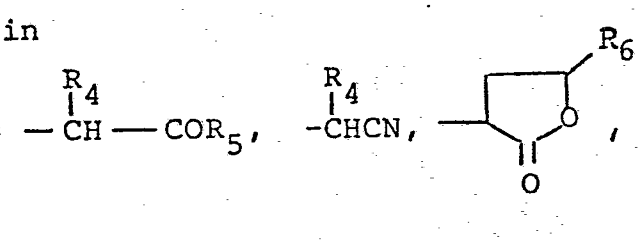

$R_1$ = $(C_1 - C_3)$-Alkyl, Halogen, $(C_1 - C_3)$-Alkoxy, $(C_1 - C_2)$-Alkylthio, $(C_2 - C_4)$-Alkenyl,

$R_2$ = Wasserstoff, $(C_1 - C_2)$-Alkyl,

$R_3$ = Methyl, Halogen,

$R_4$, $R_6$ = Wasserstoff, Methyl,

$R_5$ = Hydroxy, $(C_1 - C_4)$-Alkoxy, $(C_1 - C_3)$-Alkylthio, $(C_1 - C_2)$-Alkoxyäthoxy, Amino, $(C_1 - C_2)$-Alkylamino, Di-$(C_1 - C_2)$-alkylamino, O-Kat, wobei Kat für ein Kationenäquivalent einer anorganischen oder organischen Base steht, vorzugsweise Na, K, 1/2 Ca, Ammonium,

Het = ein 5 - 6 gliedriger, gesättigter oder ungesättigter Heterocyclus, gegebenenfalls benzokondensiert, mit bis zu 4 Heteroatomen aus der Gruppe N, O, S, vorzugsweise ein Heterocyclus aus der nachfolgend genannten Reihe:

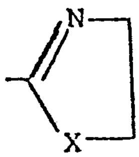 , 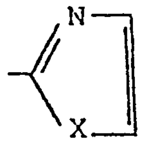 , 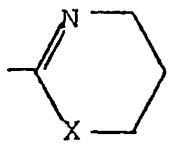 , 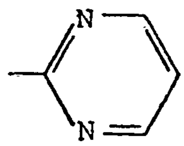 ,

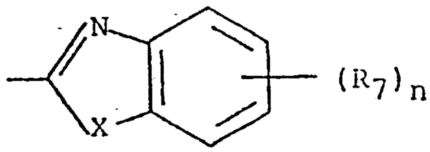 , 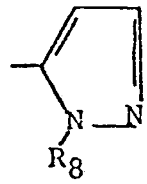 , 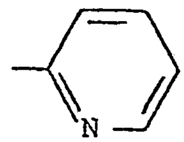 ,

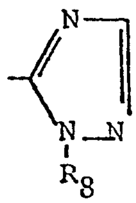 , 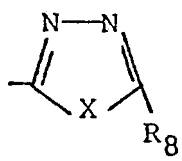 , 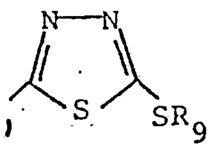 , 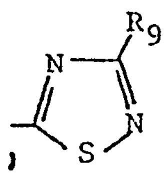 ,

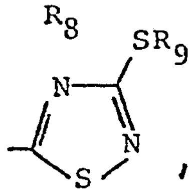 ,

wobei jeder Heterocyclus zusätzlich im Kohlenstoffteil eine oder mehrere $(C_1-C_4)$-Alkylgruppen tragen kann, und

X = O, S, $NR_8$,

$R_8$= Wasserstoff, $(C_1 - C_4)$-Alkyl,

$R_7$= $(C_1 - C_4)$-Alkyl, Halogen,

$R_9$= $(C_1 - C_4)$-Alkyl,

n = 0, 1,

m = 0, 1, 2,

q = 0, 1, 2

bedeuten,

dadurch gekennzeichnet,

I. daß man zur Herstellung von Verbindungen der Formel I, worin q= 0 bedeutet,

a) Verbindungen der allgemeinen Formel II,

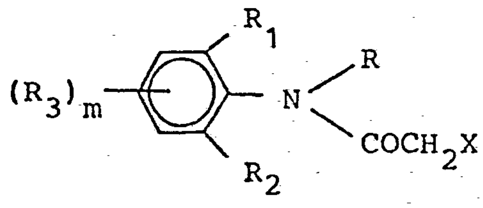

(II)

worin R, $R_1$, $R_2$, $R_3$ und m die Bedeutung wie in Formel I haben und X eine nukleophil substituierbare Abgangsgruppe, vorzugsweise aus der Gruppe Halogen, Tosylat oder Mesylat, insbesondere Chlor oder Brom, bedeutet, mit Heterocyclen der allgemeinen Formel III,

HS-Het (III)

in der Het die Bedeutung wie in Formel I hat, umsetzt, gegebenenfalls unter Zusatz von säurebindenden Mitteln, vorzugsweise in inerten Lösungs- oder Verdünnungsmitteln und bevorzugt bei Temperaturen zwischen 20 und 160°C,

oder

b) daß man Thioglykolsäureanilide der allgemeinen Formel IV,

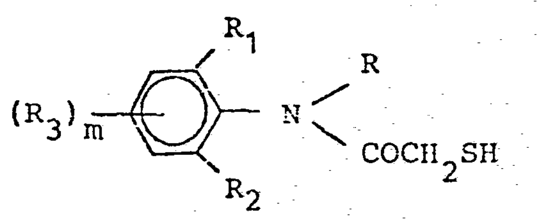

(IV)

in der R, $R_1$, $R_2$, $R_3$ und m die Bedeutungen wie in Formel I haben, mit Heterocyclen der allgemeinen Formel V,

X-Het (V)

worin Het die Bedeutung wie in Formel I hat und X eine nukleophil substituierbare Abgangsgruppe, vorzugsweise aus der Gruppe Halogen, Tosylat oder Mesylat, insbesondere Chlor oder Brom, bedeutet, umsetzt, gegebenenfalls unter Zusatz von säurebindenden Mitteln, vorzugsweise in inerten Lösungs- oder Verdünnungsmitteln und bevorzugt bei Temperaturen zwischen 20 und 160°C,

oder

c) daß man Anilinderivate der allgemeinen Formel VII,

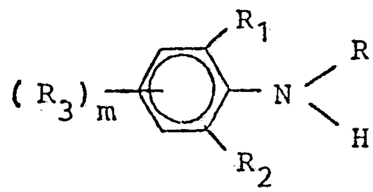

(VII)

worin R, $R_1$, $R_2$, $R_3$ und m die Bedeutungen wie in Formel I haben, mit Thioglykolsäurechloriden der allgemeinen Formel VI,

$$Cl-\overset{O}{\overset{\|}{C}}-CH_2-S-Het \qquad (VI)$$

in der Het die Bedeutung wie in Formel I hat, umsetzt, gegebenenfalls unter Zusatz von säurebindenden Mitteln, vorzugsweise in inerten Lösungs- oder Verdünnungsmitteln und bevorzugt bei Temperaturen zwischen 0 und 140°C,

und

II. daß man zur Herstellung von Verbindungen der Formel I, worin q = 1 oder 2 bedeutet, Verbindungen der Formel I, worin q = 0 bedeutet, mit entsprechenden Mengen eines Oxidationsmittels entweder zu Verbindungen der Formel I, in der q = 1 bedeutet, oder zu Verbindungen der Formel I, in der q = 2 bedeutet, umsetzt, vorzugsweise jeweils in Lösungs- oder Verdünnungsmitteln und bevorzugt bei Temperaturen zwischen -40 und 60°C für Verbindungen der Formel I mit q = 1, bzw. zwischen -20 und 160°C für Verbindungen der Formel I mit q = 2.

2. Fungizide Mittel, gekennzeichnet durch einen Gehalt an einer Verbindung der Formel I gemäß Anspruch 1 als Wirkstoff.

3. Fungizide Mittel, gekennzeichnet durch einen Gehalt von 2 bis 95 Gew.-%, vorzugsweise 5 bis 90 Gew.-% an einer Verbindung der Formel I gemäß Anspruch 1 als Wirkstoff sowie üblichen Formulierungshilfsmitteln.

4. Verwendung von Verbindungen der Formel I gemäß Ansprüchen 1, 2 und 3 zur Schädlingsbekämpfung im Pflanzenschutz.

5. Verfahren zur Bekämpfung von Schadpilzen, dadurch gekennzeichnet, daß man die von ihnen befallenen Flächen, Pflanzen oder Substrate mit einer fungizid wirksamen Menge von Wirkstoffen der Formel I gemäß Ansprüchen 1, 2 und 3 in Kontakt bringt.